(19) Europäisches Patentamt
European Patent Office
Office européen des brevets

(11) Publication number: **0 334 685 B1**

(12) **EUROPEAN PATENT SPECIFICATION**

(45) Date of publication of patent specification: **19.01.94**

(51) Int. Cl.⁵: **C07K 7/00**, A61K 37/02

(21) Application number: **89303065.0**

(22) Date of filing: **28.03.89**

(54) **Bradykinin analogues, their synthesis and their use in therapy.**

(30) Priority: **25.03.88 US 173311**

(43) Date of publication of application:
**27.09.89 Bulletin 89/39**

(45) Publication of the grant of the patent:
**19.01.94 Bulletin 94/03**

(84) Designated Contracting States:
**AT BE CH DE ES FR GB GR IT LI LU NL SE**

(56) References cited:
**EP-A- 0 257 742**

**TETRAHEDRON, vol. 44, no. 3, 1988, Pergamon Journals Ltd., Oxford (GB); T.K. SAWYER et al., pp. 661-673&NUM;**

**PEPTIDES, PROCEEDINGS OF THE TENTH AMERICAN PEPTIDE SYMPOSIUM, St. Louis, MO (US), 23-28 May 1987, ESCOM, Leiden (NL); J.M. STEWART et al., pp. 433-437&NUM;**

**EUROPEAN JOURNAL OF PHARMACOLOGY, vol. 115, 1988, Elsevier Science Publishers B.V. (Biomedical Division); G. DRAPEAU et al., pp. 193-195&NUM;**

(73) Proprietor: **THE ADMINISTRATORS OF THE TULANE UNIVERSITY EDUCATIONAL FUND**
**1430 Tulane Avenue,**
**Tulane University**
**New Orleans, Louisiana 70112-2699(US)**

(72) Inventor: **Coy, David H.**
**4319 Perrier Street**
**New Orleans, LA 70115(US)**
Inventor: **Moreau, Jacques-Pierre**
**159 Westboro Road**
**Upton, MA 01568(US)**
Inventor: **Taylor, John E.**
**74 Fisk Mill Road**
**Upton, MA 01568(US)**

(74) Representative: **Deans, Michael John Percy et al**
**Lloyd Wise, Tregear & CO.**
**Norman House**
**105-109 Strand**
**London WC2R OAE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to bradykinin analogues.

Martinez et al. observed in studies on the mode of action of gastrin that changes in the peptide backbone (replacement of a carbonyl group with a methylene group) could provide analogues to gastrin with significantly altered biological activity, as agonists or antagonists (Martinez et al., J. Med. Chem., 28: 1874-1879 (1985)). Yet peptide bond replacement does not systematically provide for antagonist activity as Sasaki et al. found in a study of somatostatin analogues (Sasaki et al., J. Med. Chem., 30:1162-1166 (1987)-), and Martinez et al. discovered in a study on the effects of changes to peptide bond of the C-terminal heptapeptide of cholecystokinin, a peptide which has much the same biological activity and the same final four amino acids as gastrin (Rodriquez et al., J. Med. Chem.,
30:1366-1373 (1987)).

Abbreviations (uncommon):

pGlu =

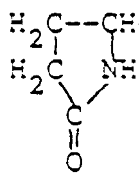 (pyroglutamate);

Nle =

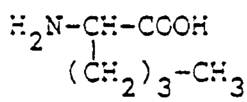 (norleucine)

| | |
|---|---|
| Pal = | 3-pyridyl-alanine |
| Nal = | naphthylalanine |
| HYP = | hydroxyproline |

In general, the invention features a bradykinin analog of the formula (1):

$Q^0$-$A^1$-$A^2$-$A^3$-Gly-$A^5$-$A^6$-$A^7$-$A^8$-$A^9$-$Z^{10}$

wherein

| | |
|---|---|
| $Q^0$ = | L-Arg, D-Arg, L-homo-Arg, H, D-homo-Arg, L-Lys, D-Lys, lower (1-5 carbon atoms) Σ-N-alkyl-L-Lys, lower Σ-N-alkyl-D-Lys, lower Σ-N-alkyl-L-His, lower Σ-N-alkyl-D-His, lower Σ-N-alkyl-D-His, lower Σ-N-alkyl-L-Pal, lower Σ-N-alkyl-D-Pal, lower acyl, or lower α-N-alkyl; |
| $A^1$ and $A^9$ (independently) = | Arg, homo-Arg, Lys, lower Σ-N-alkyl-Lys, His, or Pal; |
| $A^2$ = | Pro, hydroxy-Pro, or N-Me-Ala; |
| $A^3$ = | Pro, hydroxy-Pro, or N-Me-Ala; |
| $A^5$ and $A^8$ (independently) = | Phe, thienylalanine, His, Trp, Nal, Pal, or P-X-Phe (X = F, Cl, Br, OH, or $CH_3$); |
| $A^6$ = | Ser, Thr, Ala, Leu, Ile, Val, or Tyr; |
| $A^7$ = | Pro, hydroxy-Pro, N-Me-Ala, D-Phe, or D-thienylalanine; |
| $Z^{10}$ = | OH, COOH, $NH_2$, or lower alkylamide; |

provided that,

for each of the residues $A^5$, $A^6$, $A^7$, and $A^8$, independently, the carbon atom participating in the amide bond between that residue and the nitrogen atom of the alpha amino group of the adjacent amino acid residue may be a carbonyl carbon or may be reduced to a methylene carbon, provided that at least one such carbon atom must be reduced to a methylene carbon (i.e., at least one of the subject peptide CONH bonds must be replaced by a non-peptide $CH_2NH$ bond); or a pharmaceutically acceptable salt thereof.

Preferred bradykinin analogs of the invention have non-peptide bonds joining residue $A^8$ to $A^9$, or $A^5$ to $A^6$. (In the examples given below, non-peptide bonds are symbolized by "$\psi[CH_2NH]$".)

Preferred analogs are:

Arg-Pro-Pro-Gly-Phe-ψ[$CH_2$NH]-Ser-Pro-Phe-Arg; and Arg-Pro-Pro-Gly-Phe-Ser-Pro-Phe-ψ[$CH_2$NH]-Arg-OH.

The present bradykinin analogues, which act as bradykinin antagonists are useful for preventing increased vascular permeability that occurs during non-infectious inflammation, as analgesics, for preventing edema due to pulmonary brain trauma, in preventing shock due to hemorrhage, or for any condition characterized by vasodilation-induced swelling and/or itching.

The invention also provides a method for synthesizing a bradykinin peptide analogue of formula (1), said method comprising providing an amino terminal amino acid attached to a resin support and coupling successive amino acids to said amino terminal amino acid to produce said peptide analog, removing said peptide analog from said resin, and purifying said peptide analog.

Other features and advantages of the invention will be apparent from the following description of the preferred embodiments thereof, and from the claims.

We now describe the structure, synthesis, and use of the preferred embodiments of the invention.

The bradykinin analogs of the invention all have a non-peptide bond in at least one of the indicated positions. By non-peptide bond is meant that the carbon atom participating in the bond between two residues is reduced from a carbonyl carbon to a methylene carbon. The peptide bond reduction method which yields this non-peptide bond is described in Coy et al., U.S. Patent No. 4,803,261, the disclosure of which is hereby incorporated by reference.

These bradykinin analogues can be provided in the form of pharmaceutically acceptable salts. Examples of preferred salts are those with therapeutically acceptable organic acids, e.g., acetic lactic, maleic, citric, malic, ascorbic, succinic, benzoic, salicylic, methanesulfonic, toluenesulfonic, or pamoic acid, as well as polymeric acids such as tannic acid or carboxymethyl cellulose, and salts with inorganic acids such as the hydrohalic acids, e.g., hydrochloric acid, sulfuric acid, or phosphoric acid.

The synthesis of Boc-Arg(tosyl)Pro-Pro-Gly-Phe-ψ[$CH_2$NH]-Ser(benzyl)-Pro-Phe-Arg(nitro)-O-resin is carried out as follows.

Boc-Arg(nitro)-polystyrene resin (Vega Biochemicals) (0.86 gm, 0.5 mmole) is placed in the reaction vessel of an Advanced ChemTech ACT 200 peptide synthesizer programmed to perform the following reaction cycle; (a) methylene chloride wash; (b) 33% trifluoroacetic acid in methylene chloride (2 times for 1 and 25 min each); (c) methylene chloride wash; (d) 10% triethylamine in dimethyformamide; (e) methylene chloride wash.

The neutralized resin is stirred with Boc-Phe and diisopropylcarbodiimide (1.5 mmole each) in methylene chloride for 1 h and the resulting amino acid resin is then cycled through steps (a) to (e) in the above wash program. The Boc group is then removed by TFA treatment and the following amino acid derivatives (1.5 mmole) are then coupled successively by the same procedure: Boc-Phe, Boc-Pro, Boc-Ser-(benzyl). Boc-Phe aldehyde (1.5 mmoles), prepared by the method of Fehrentz and Castro, Synthesis, p. 676 (1983) is dissolved in 5 ml of dry DMF and added to the resin TFA salt suspension followed by the addition of 400 mg (8 mmoles) of sodium cyanoborohydride (Coy et al., U.S. Patent No. 4,803,261). After stirring for 1 h, the resin mixture is found to be negative to ninhydrin reaction (1 min) indicating complete derivitization of the free amino group.

After removal of the Boc group, Boc-Gly-p-nitrophenyl ester (3.0 mmoles) is then coupled in dimethylformamide. The following amino acids (1.5 mmole) are then coupled successively by the carbodiimide procedure: Boc-Pro, Boc-Pro, Boc-Arg(nitro). After drying, the peptide resin weighs 1.23 g.

The synthesis of H-Arg-Pro-Pro-Gly-Phe-ψ [$CH_2$NH]-Ser-Pro-Phe-Arg-OH follows.

The resin, as described above, (1.23 g, 0.5 mmole) is mixed with anisole (5 ml) and anhydrous hydrogenfluoride (35 ml) at 0 °C and stirred for 45 min. Excess fluoride is evaporated rapidly under a stream of dry nitrogen and free peptide precipitated and washed with ether. The crude peptide is dissolved in a minimum volume of 2M acetic acid and eluted on a column (2.5 x 95 cm) of Sephadex G-25. Fractions containing a major component by uv absorption (254 nm) and thin layer chromatography are then pooled, evaporated to a small volume and applied to a column (1.5 x 50 cm) of Vydac octadecylsilane (10-15μM).

The peptide is eluted with a linear gradient of 10-35% acentonitrile in 0.1% trifluoroacetic acid in water. Fractions are examined by thin layer chromatography and analytical high performance liquid chromatography and pooled to give maximum purity. Repeated lyophilization of the solution from water gives 104 mg of the product as a white,fluffy powder.

The product is found to be homogeneous by hplc and tlc. Amino acid analysis of an acid hydrolysate confirms the composition of the octapeptide. The presence of the Pheψ[$CH_2$NH] peptide bond is also demonstrated by fast atom bombardment mass spectrometry.

Other peptides can be prepared in similar yields in an analogous fashion by appropriately modifying the above procedure.

A linear bradykinin analog can be tested for effectiveness as either an agonist or antagonist of bradykinin using the following method.

Bradykinin-Stimulated Cyclic GMP Formation

Described below is a method that employs cultured neuroblastoma cells, which have high-affinity receptor sites for bradykinin and respond to bradykinin with an increase in intracellular levels of cyclic GMP.

Murine neuroblastoma cells (clone NIE-115) were cultured in Dulbecco's modified Eagle's medium without antibiotics and supplemented with 10% (vol/vol) fetal calf serum. Cells were grown in wells of a 24 multiwell plastic tray in an atmosphere of 10% $CO_2$-90% air at 37°C.

Cell Culture

Described below is an assay method that employs cultured neuroblastoma cells, which have high-affinity receptor sites for bradykinin and respond to bradykinin with an increase in intracellular levels of cyclic GMP. Mouse neuroblastoma cells (Clone NIE-115) were obtained from Dr. Elliott Richelson (Mayo Clinic and Foundation, Rochester, MN, Snider and Richelson, 1984, J. Neurochem. 43:1749-1754) and cultured in Dulbecco's modified Eagle's medium (Sigma Chem., St. Louis, MO) without antibiotics and supplemented with 10% fetal calf serum (Sigma Chem.). The stock cultures were grown in an atmosphere of 10% $CO_2$/90% humidified air.

Bradykinin-Stimulated cyclic GMP Formation

The assay of cyclic GMP formation was modified from the procedure described by Snider and Richelson (1984). The cells were initially grown for five days in 24-well culture plates. On the day of assay the culture medium was then removed, and the cells were washed twice with a phosphate-buffered saline solution (PBS) containing 110 mM NaCl, 5.3 mM KCl, 1.8 mM $CaCl_2$, 1.0 mM $MgCl_2$, 2.0 mM $Na_2HPO^4$, 25 mM glucose, and 70 mM sucrose (pH 7.4, 335-340 mmol). The cells were labeled with [$^3$H]guanine (4 $\mu$Ci/ml, 0.6 $\mu$M) in PBS for 45 min at 37°C. After the [$^3$H]guanine incubation the PBS/[$^3$H]guanine solution was removed and each well was washed an additional time with PBS. PBS (240 $\mu$l) and antagonists (30 $\mu$l) were added, and the cells were pre-incubated for 10 min (37°C). To assay for agonist activity, either Bradykinin or the test compound (30 $\mu$l) (Bachem Inc., Torrence, CA) was added, and the incubation was continued for an addtional 30 sec. (37 C). To test for antagonist activity, labeled cells were preincubated for 5 min. with the test antagonist before the addition of Bradykinin (indicated as "+" in results of antagonist assays, below). The Bradykinin or test compound stimulation was terminated by the addition of 30 $\mu$l of 50% (w/v) trichloroacetic acid, and the contents of each well were transferred to (0.8 x 8.0 cm) AG50 W-X2 ion exchange column which had been equilibrated with 0.1 N HCl. The columns were then sequentially washed with 4.4 ml of 0.1 N HCl (eluate discarded), 1.0 ml $H_2O$ (eluate discarded), and 1.5 ml $H_2O$ which was collected into 12 x 75 mm plastic culture tubes. To this last eluate, equal volumes (30 $\mu$l) of 2.67 M $ZnSO_4$ and 2.67 M $Na_2CO_3$ were added to further precipitate any residual [$^3$H]GDP or [$^3$H]GTP. After the precipitate had been removed by centrifugation, the supernatant was transferred to 7 ml of Scint A (Packard) and the radioactivity determined by liquid scintillation spectrometry.

[$^3$H]Bradykinin Binding Experiments

Membranes from the cells were prepared by homogenizing the NIE-115 cells in ice-cold buffer with a Brinkman Polytron (seting 6, 15 sec) and centrifuging twice (39,000 g, 10 min.) with an intermediate resuspension in fresh buffer. Aliquots of the membrane preparation were incubated with 1.0 nM (competition experiments) or 0.3 - 5 nM (saturation experiments) [$^3$H]bradykinin (88.7 Ci/mmol, New England Nuclear) for 90 min (25°C) in a final volume of 1.0 ml. The binding assay was terminated by rapid filtration through Whatman GF/B filters that had previously soaked in 0.1% aqueous polyethyleneimine. Each assay tube and filter were immediately washed three times with 5 ml aliquots of ice-cold buffer, and the bound radioactivity trapped on the filters were counted by liquid scintillation spectrometry.

As shown in Table I, incubation of $N_IE$-115 cells with bradykinin produced a dose-dependent stimulation of cyclic GMP formation. Maximum stimulation of cyclic GMP formation (to ~ 6 pmoles) occurred at approximately 100 nM of bradykinin.

Results of Assays of Test Peptides

A true antagonist molecule binds to a specific receptor (i.e. the Bradykinin receptor) with the same affinity as an agonist but lacks biological activity.

An example of a true antagonist is BIM-31006, as shown below. (Where bradykinin analog numbers are also referred to in Table I, amino acid formulas are also given in the table.)

Assay for antagonist activity of BIM-31006

| Condition | cGMP response (dpm) |
|---|---|
| basal | 327 |
| bradykinin (3 nM) | 2459 |
| +BIM-31006 (1 nM) | 2004 |
| +BIM-31006 (10 nM) | 1201 |
| +BIM-31006 (100 nM) | 418 |

Assay for agonist activity of BIM-31006

| Condition | cGMP response (dpm) |
|---|---|
| basal | 437 |
| BIM-31006 (0.3 nM) | 424 |
| BIM-31006 (1.0 nM) | 335 |
| BIM-31006 (3.0 nM) | 448 |
| BIM-31006 (10 nM) | 266 |
| BIM-31006 (30 nM) | 333 |
| BIM-31006 (100 nM) | 427 |
| bradykinin (3 nM) | 3019 |

The results show that BIM-31006 does have antagonist activity in the concentration range of 1 - 100 nM, and does not have agonist activity in the same concentration range.

A molecule may appear to be an antagonist, but not be a true antagonist, if it acts as a superagonist by causing over-stimulation of its target receptor and thus desensitizes the receptor to stimulation. This is a mechanism by which a molecule that is a superagonist may appear to be an antagonist, An example of this type of pseudo-antagonist is

D-Arg$^0$,HYP$^3\psi[CH_2NH]$Phe$^8$BK, or BIM-31012.

BIM-31012 is an example of an antagonist which acts by desensitization of the receptor. The antagonist and agonist activities was shown in the following experiment:

Assay for antagonist activity of BIM-31012

| Condition | cGMP response (dpm) |
|---|---|
| basal | 279 |
| bradykinin (3 nM) | 1929 |
| +BIM-31012 (0.1 nM) | 1818 |
| +BIM-31012 (1 nM) | 516 |
| +BIM-31012 (10 nM) | 401 |
| +BIM-31012 (100 nM) | 296 |

Therefore, in concentrations between 1 - 100 nM, BIM-31012 is an antagonist.

Assay for agonist activity of BIM-31012

| Condition | cGMP response (dpm) |
|---|---|
| basal | 279 |
| BIM-31012 (1 nM) | 1379 |
| BIM-31012 (10 nM) | 4608 |
| BIM-31012 (100 nM) | 5938 |

These results show that, in the same concentration range, BIM-31012 is also an agonist. To determine if the apparent antagonistic activity of BIM-31012, shown above, was a result of agonist-induced receptor desensitization, the cells were preincubated with bradykinin for 5 min. at 37°C before assaying for Bradykinin-stimulated cGMP formation.

| Condition | cGMP response (dpm) |
|---|---|
| basal | 385 |
| bradykinin (3 nM) | 2085 |
| +bradykinin (1 nM) | 1655 |
| +bradykinin (10 nM) | 1591 |
| +bradykinin (100 nM) | 496 |
| +bradykinin (1000 nM) | 348 |

The results demonstrate that pre-incubation of cells with bradykinin results in apparent antagonistic activity for bradykinin itself, which is normally an agonist. This suggests that the initial exposure to bradykinin over-stimulated the receptor and thus desensitized it, which also explains the apparent antagonism of BIM-31012.

Other bradykinin analogs may be mixed antagonist/agonists; i.e., may be true antagonists over a given concentration range, but agonists at a different concentration range. BIM-31005, $Phe^8\psi[CH_2NH]BK$ may also be a mixed antagonist/agonist, as shown by the following experiments.

Assay for agonist activity of BIM-31005

| Condition | cyclic GMP response (dpm) |
|---|---|
| basal | 265 |
| BIM-31005 (0.1 nM) | 246 |
| BIM-31005 (0.3 nM) | 260 |
| BIM-31005 (1.0 nM) | 306 |
| BIM-31005 (3.0 nM) | 227 |
| BIM-31005 (10 nM) | 1481 |
| BIM-31005 (30 nM) | 3705 |
| Bradykinin (3.0 nM) | 3821 |

The results show tht BIM-31005 is less potent than Bradykinin as an agonist. BIM-31005, however, binds with higher affinity to the receptor than Bradykinin. The Ki for BIM-31005 is 0.38 nM, and for bradykinin is 1.0 nM. Table I and the assay below show antagonist activity of BIM-31005.

| Condition | cyclic GMP response (dpm) |
|---|---|
| basal | 327 |
| bradykinin (3.0 nm) | 2594 |
| +BIM-31005 (1.0 nM) | 2459 |
| +BIM-31005 (10 nM) | 1390 |
| +BIM-31005 (100 nM) | 538 |

These results suggest that the antagonistic activity of BIM-31005 begins to occur at 1.0 nM; at this concentration, no agonist activity is observed. This suggests a mxture of agonist/antagonist activities. BIM-31005 may also act antagonistically by desensitizing the receptor at those concentrations at which it

appears to act as an antagonist.

Another example of a mixed antagonist/agonist is D-$Arg^0$,$HYP^3\psi[CH_2NH]Phe^5$BK, or BIM-31011. The results for the BIM-31011 analog are shown by the following experiment:

Assay for antagonist activity of BIM-31011

| Condition | cGMP response (dpm) | %inhibition |
|---|---|---|
| basal | 308 | -- |
| Bradykinin | 6968 | -- |
| +BIM-31011(1 nm) | 4757 | 33 |
| (10 nm) | 4619 | 35 |
| (100 nm) | 2886 | 61 |
| (1000 nm) | 320 | 99 |

Assay for agonist activity of BIM-31011

| Condition | cGMP response (dpm) |
|---|---|
| basal | 308 |
| BIM-31011(0.1 nm) | 238 |
| (1.0 nm) | 325 |
| (10 nm) | 314 |

This compound shows antagonist activity in the range of 1 - 1000 nM and no agonist activity up to 10 nM.

The sharp drop in antagonist activity seen above between 100 nM and 1000 nM was investigated further.

Assay for antagonist activity of BIM-31011

| Condition | cGMP response (dpm) | %inhibition |
|---|---|---|
| basal | 102 | |
| bradykinin (3 nM) | 1882 | |
| +BIM-31011 (100 nM) | 1632 | |
| +BIM-31011 (300 nM) | 852 | |
| +BIM-31011 (1000 nM) | 166 | |

Assay for agonist activity of BIM-31011

| Condition | cGMP response (dpm) |
|---|---|
| basal | 179 |
| BIM-31011 (100 nM) | 146 |
| BIM-31011 (300 nM) | 281 |
| BIM-31011 (1000 nM) | 1987 |
| bradykinin (3 nM) | 1336 |

The results show that antagonist activity of BIM-31011 starts to decrease at a concentration of 300 nM and completely disappears at 1000 nM, whereas agonist activity is markedly stimulated at 1000 nM. BIM-31011 is therefore a mixed agonist/antagonist.

Inhibition of bradykinin-stimulated cyclic GMP formation (to ~ 2.4 pmoles) by the bradykinin analogues, $Phe^5\psi[CH_2NH]$-bradykinin (BIM-31002) and $Phe^8\psi(CH_2NH)$-bradykinin (BIM-31005) occurred at approximately 3 - 10 nM, as shown in Table I. The $Phe^5$- and $Phe^8$-$\psi[CH_2NH]$-bradykinin antagonists are as potent as other analogues, such as those developed by Schachter et al., 1987, Br. J. Pharmac. 92:851-855; Rifo et al., 1987, Eur. J. Pharmac. 142:305-312; Vavrek and Stewart, 1985, Peptides 6:161-164; and Steranka et al., 1987, Eur. J. Pharmac, 136:261-262). In addition, Drapeau et al., 1988, Eur. J. Pharm. 155:193, reported that

$[Phe^8\psi[CH_2NH]Arg^9]BK$ is a very potent agonist.

Table I

| Bradykinin Antagonists - Receptor Binding and Biological Activity | | | |
|---|---|---|---|
| Compound | Structure | Receptor Binding Ki (nM)* | cyclic GMP Antagonism Ki (nM)** |
| BIM-31002 | $Phe^5\psi[CH_2NH]BK$ | 1.0 ± 0.36 | 8.4 ± 3.8 |
| BIM-31003 | $Gly^4\psi[CH_2NH]BK$ | 2215 ± 298 | >10000 |
| BIM-31004 | $Pro^7\psi[CH_2NH]BK$ | 11 ± 1.3 | 97 ± 56 |
| BIM-31005 | $Phe^8\psi[CH_2NH]BK$ | 0.38 ±0.07 | 9.1 ± 2.1 |
| BIM-31006 | $[D\text{-}Arg^0,Hyp^3, D\text{-}Phe^7]BK$ | 0.24 ±0.05 | 14 ± 1.7 |
| BIM-31007 | $Pro^3\psi[C_2NH]BK$ | 48 ± 8.6 | >10000 |
| BIM-31008 | $Lys^{1,9},Phe^8\ \psi[CH_2NH]BK$ | 2294 | >10000 |
| BIM-31009 | $Phe^{5,8}\psi[CH_2NH]BK$ | >10000 | >10000 |

*Inhibition of 1.0 nM[$^3$H]bradykinin binding to mouse neuroblastoma cells (clone NIE-115)
**Inhibition of bradykinin (30 nM) - stimulated cyclic GMP formation in mouse neuroblastoma cells (clone NIE-115).

The described bradykinin analogues may be administered to a mammal, particularly a human, in one of the traditional modes (e.g., orally, parenterally, transdermally, or transmucosally), in a sustained release formulation using a biodegradable biocompatible polymer, or by on-site delivery using micelles, gels and liposomes.

The bradykinin analogues which act as antagonists are suitable for the treatment of medical disorders characterized by unwanted vascular dilation or permeability, e.g., common cold symptoms, vascular permeability-induced pain, edema caused by brain trauma, hemorrhage-induced shock, poison ivy, or other non-infectious swelling or itching, and are also suitable for the treatment of pain and arthritis. Bradykinin agonists can be used to treat hypertension, or to increase blood flow e.g., caused by poor circulation in the extremities. The bradykinin analogues can be administered (most preferably, topically) to a human patient in a dosage of 0.5µg/kg/ day to 5 mg/kg/day, preferably 10-1000 µg/kg/day.

## Claims

**1.** A bradykinin analog of the formula:

$Q^0\text{-}A^1\text{-}A^2\text{-}A^3\text{-Gly-}A^5\text{-}A^6\text{-}A^7\text{-}A^8\text{-}A^9\text{-}Z^{10}$

wherein

| | |
|---|---|
| $Q^0$ = | L-Arg, D-Arg, L-homo-Arg, H, D-homo-Arg, L-Lys, D-Lys, lower (1-5 carbon atoms) Σ-N-alkyl-L-Lys, lower Σ-N-alkyl-D-Lys, lower Σ-N-alkyl-L-His, lower Σ-N-alkyl-D-His, lower Σ-N-alkyl-L-Pal, lower Σ-N-alkyl-D-Pal, lower acyl, or lower α-N-alkyl; |
| $A^1$ and $A^9$ (independently) = | Arg, homo-Arg, Lys, lower Σ-N-alkyl-Lys, His, or Pal; |
| $A^2$ = | Pro, hydroxy-Pro, or N-Me-Ala; |
| $A^3$ = | Pro, hydroxy-Pro, or N-Me-Ala; |
| $A^5$ and $A^8$ (independently) = | Phe, thienylalanine, His Trp, Nal, Pal, or P-X-Phe, (X = F, Cl, Br, OH, or $CH_3$); |
| $A^6$ = | Ser, Thr, Ala, Leu, Ile, Val, or Tyr; |
| $A^7$ = | Pro, hydroxy-Pro, N-Me-Ala, D-Phe, or D-thienylalanine; |
| $Z^{10}$ = | OH, COOH, $NH_2$, or lower alkylamide; |

provided that,

for each of the residues $A^5$, $A^6$, $A^7$, and $A^8$, independently, the carbon atom participating in the amide bond between that residue and the nitrogen atom of the alpha amino group of the adjacent amino acid residue may be a carbonyl carbon or may be reduced to a methylene carbon, provided that at least one such carbon atom must be reduced to a methylene carbon; or a pharmaceutically acceptable salt thereof.

2. The bradykinin analogue or its salt of Claim 1, wherein, for each of the residues $A^5$ or $A^8$, independently, the carbon atom participating in the amide bond between that residue and the nitrogen atom of the alpha amino group of the adjacent amino acid residue may be a carbonyl carbon or may be reduced to a methylene carbon, provided that at least one such carbon atom must be reduced to a methylene carbon.

3. The bradykinin analog of Claim 1, having the amino acid formula:
Arg-Pro-Pro-Gly-Phe-Ser-Pro-Phe-$\psi$[$CH_2$NH]-Arg-OH, or a pharmaceutically acceptable salt thereof.

4. The bradykinin analog of Claim 1, having the formula:
Arg-Pro-Pro-Gly-Phe-$\psi$[$CH_2$NH]-Ser-Pro-Phe-Arg-OH, or a pharmaceutically acceptable salt thereof.

5. A method for synthesizing a bradykinin peptide analog as defined in any of Claims 1 to 4, said method comprising providing an amino terminal amino acid attached to a resin support and coupling successive amino acids to said amino terminal amino acid to produce said peptide analog, removing said peptide analog from said resin, and purifying said peptide analog.

6. A bradykinin analogue or its salt according to any of Claims 1 to 4 for use in therapy.

7. A bradykinin analogue or its salt according to any of Claims 1 to 4 for use in the relief of conditions characterised by unwanted vascular dilation or permeability, or for the treatment of pain or arthritis.

8. A bradykinin analogue or its salt according to any of Claims 1 to 4 for use in the treatment of hypertension, poor circulation, or in any condition where increased blood flow is required.

9. Use of a bradykinin analogue or its salt according to any of Claims 1 to 4 for the manufacture of a medicament for use in the relief of conditions characterised by unwanted vascular dilation or permeability, or for the treatment of pain or arthritis.

10. Use of a bradykinin analogue or its salt according to any of Claims 1 to 4 for the manufacture of a medicament for use in the treatment of hypertension, poor circulation, or in any condition where increased blood flow is required.

## Patentansprüche

1. Bradylinin-Analogon der Formel

$Q^0$-$A^1$-$A^2$-$A^3$-Gly-$A^5$-$A^6$-$A^7$-$A^8$-$A^9$-$Z^{10}$,

worin

| | |
|---|---|
| $Q^0$ = | L-Arg, D-Arg, L-homo-Arg, H, D-homo-Arg, L-Lys, D-Lys ; niedrig (1-5 Kohlenstoffatome)<br>Σ-N-Niedrigalkyl-L-Lys,<br>Σ-N-Niedrigalkyl-D-Lys,<br>Σ-N-Niedrigalkyl-L-His,<br>Σ-N-Niedrigalkyl-D-His,<br>Σ-N-Niedrigalkyl-L-Pal,<br>Σ-N-Niedrigalkyl-D-Pal,<br>Niedrigacryl oder α-N-Niedrigalkyl ;<br>a-N-Alkyl |
| $A^1$ and $A^9$ (unabhängig) = | Arg, homo-Arg, Lys, Σ-N-Niedrigalkyl-Lys, His, oder Pal; |
| $A^2$ = | Pro, hydroxy-Pro, oder N-Me-Ala; |
| $A^3$ = | Pro, hydroxy-Pro, oder N-Me-Ala; |
| $A^5$ and $A^8$ (unabhängig) = | Phe, Thienylalanin, His, Trp, Nal, Pal, oder P-X-Phe (X = F, Cl, Br, OH, oder $CH_3$); |
| $A^6$ = | Ser, Thr, Ala, Leu, Ile, Val, oder Tyr ; |
| $A^7$ = | Pro, hydroxy-Pro, N-Me-Ala, D-Phe oder D-Thienylalanin ; |
| $Z^{10}$ = | OH, COOH, $NH_2$, oder Niedrigalkylamid; |

mit der Maßgabe, daß
für jeden der Reste $A^5$, $A^6$, $A^7$ und $A^8$ unabhängig das Kohlenstoffatom, das Teil einer Amidbindung zwischen diesem Rest und dem Stickstoffatom der alpha-Aminogruppe des benachbarten Aminosäure-Restes ist, ein Carbonyl-Kohlenstoff sein kann oder zu einer Methylengruppe reduziert werden kann, mit der Maßgabe, daß mindestens ein solches Kohlenstoffatom zu einem Methylen-Kohlenstoff reduziert werden muß; oder ein pharmazeutisch verträgliches Salz davon.

**2.** Bradykinin-Analogon oder dessen Salz nach Anspruch 1, worin für jeden der Reste $A^5$ oder $A^8$ unabhängig das Kohlenstoffatom, das Teil der Amid-Bindung zwischen diesem Rest und dem Stickstoffatom der alpha-Aminogruppe des angrenzenden Aminosäurerestes ein Carbonyl-Kohlenstoff sein kann oder zu einem Methyl-Kohlenstoff reduziert werden kann, mit der Maßgabe, daß mindestens ein solches Kohlenstoffatom zu einem Methylen-Kohlenstoff reduziert werden muß.

**3.** Bradylinin-Analogon nach Anspruch 1 mit der Aminosäure-Formel :
Arg-Pro-Pro-Gly-Phe-Ser-Pro-Phe-Ψ[$CH_2$NH]-Arg-OH oder ein pharmazeutisch verträgliches Salz davon.

**4.** Bradykinin-Analogon nach Anspruch 1 mit der Formel:
Arg-Pro-Pro-Gly-Phe-Ψ[$CH_2$NH]-Ser-Pro-Phe-Arg-OH oder ein pharmazeutisch verträgliches Salz davon.

**5.** Verfahren zur Synthese eines Bradykinin-Peptidanalogons nach einem der Ansprüche 1 bis 4, wobei das genannte Verfahren umfaßt Bereitstellen einer Aminoterminalen Aminosäure, die an ein Harz-Trägermaterial gebunden ist und Kuppeln von aufeinanderfolgenden Aminosäuren an die genannte Amino-terminalen Aminosäure, um das genannte Peptid-Analogon herzustellen, Entfernen des genannten Peptid-Analogons von dem genannten Harz und Reinigen des genannten Peptid-Analogons.

**6.** Bradykinin-Analogon oder dessen Salz nach einem der Ansprüche 1 bis 4 zur therapeutischen Anwendung.

**7.** Bradykinin-Analogon oder dessen Salz nach einem der Ansprüche 1 bis 4 zur Verwendung bei der Linderung von Zuständen, die gekennzeichnet sind durch eine unerwünschte vaskuläre Dilatation oder Durchlässigkeit, oder zur Behandlung von Schmerzen oder Arthritis.

**8.** Bradykinin-Analogon oder dessen Salz nach einem der Ansprüche 1 bis 4 zur Anwendung bei der Behandlung von Bluthochdruck, schlechter Zirkulation oder von irgendeinem Zustand, bei dem ein verstärkter Blutfluß erforderlich ist.

**9.** Verwendung eines Bradykinin-Analogons oder dessen Salzes nach einem der Ansprüche 1 bis 4 zur Herstellung eines Medikaments zur Anwendung bei der Linderung von Zuständen, die gekennzeichnet sind durch eine unerwünschte vaskuläre Dilatation oder Durchlässigkeit, oder zur Behandlung von Schmerzen oder Arthritis.

**10.** Verwendung eines Bradykinin-Analogons oder dessen Salzes nach einem der Ansprüche 1 bis 4 zur Herstellung eines Medikaments zur Anwendung bei der Behandlung von Bluthochdruck, schlechter Zirkulation oder von irgendeinem Zustand, bei dem ein verstärkter Blutfluß erforderlich ist.

**Revendications**

**1.** Un analogue de la bradykinine de formule
$Q^0$-$A^1$-$A^2$-$A^3$-Gly-$A^5$-$A^6$-$A^7$-$A^8$-$A^9$-$Z^{10}$

dans laquelle

| | |
|---|---|
| $Q^0$ = | L-Arg, D-Arg, L-homo-Arg, H, D-homo-Arg, L-Lys, D-Lys, Σ-N-alkyl-L-Lys inférieur (1-5 atomes de carbone), Σ-N-alkyl-D-Lys inférieur, Σ-N-alkyl-L-His inférieur, Σ-N-alkyl-D-His inférieur, Σ-N-alkyl-L-Pal inférieur, Σ-N-alkyl-D-Pal inférieur, acyle inférieur ou α-N-alkyle inférieur; |

| | |
|---|---|
| $A^1$ et (indépendamment) $A^9$ = | Arg, homo-Arg, Lys, Σ-N-alkyl-Lys inférieur, His, ou Pal; |
| $A^2$ = | Pro, hydroxy-Pro, ou N-Me-Ala; |
| $A^3$ = | Pro, hydroxy-Pro, ou N-Me-Ala; |
| $A^5$ et (indépendamment) $A^8$ = | Phe, thiénylalanine, His, Trp, Nal, Pal, ou P-X-Phe, (X = F,Cl,Br,OH ou $CH_3$); |
| $A^6$ = | Ser, Thr, Ala, Leu, Ile, Val, ou Tyr; |
| $A^7$ = | Pro, hydroxy-Pro, N-Me-Ala, D-Phe, ou D-thiénylalanine; |
| $Z^{10}$ = | OH, COOH, $NH_2$, ou alkylamide inférieur; |

étant entendu que:

pour chacun des résidus $A^5$, $A^6$, $A^7$ et $A^8$, indépendamment, l'atome de carbone participant à la liaison amide entre ce résidu et l'atome d'azote du groupe alpha-amino du résidu amino-acide adjacent, peut être un carbone de carbonyle ou peut être réduit à un carbone de méthylène , étant entendu qu'au moins un atome de carbone de ce genre doit être réduit à un atome de méthylène: ou un sel pharmaceutiquement acceptable de ce dernier.

**2.** L'analogue de la bradykinine ou son sel selon la revendication 1, dans lequel, pour chacun des résidus $A^5$ ou $A^8$, indépendamment, l'atome de carbone-participant à la liaison amide entre ce résidu et l'atome d'azote du groupe alpha-amino du résidu amino-acide adjacent, peut être un carbone de carbonyle ou peut être réduit à un carbone de méthylène, étant entendu qu'au moins un atome de carbone de ce genre doit être réduit à un carbone de méthylène.

**3.** L'analogue de la bradykinine selon la revendication 1, ayant la formule d'amino-acide suivante :
Arg-Pro-Pro-Gly-Phe-Ser-Pro-Phe-Ψ[$CH_2$NH]-Arg-OH, ou un sel pharmaceutiquement acceptable de celui-ci.

**4.** L'analogue de la bradykinine selon la revendication 1, ayant la formule:
Arg-Pro-Pro-Gly-Phe-Ψ[$CH_2$NH]-Ser-Pro-Phe-Arg-OH, ou un sel pharmaceutiquement acceptable de celui-ci.

**5.** Une méthode pour synthétiser un peptide analogue de la bradykinine tel que défini dans l'une quelconque des revendications 1 à 4, ladite méthode comprenant la fixation d'un amino-acide amino-terminal à un support de résine et le couplage successif d'amino-acides audit amino-acide amino-terminal pour produire ledit peptide analogue, le clivage dudit peptide analogue de ladite résine et la purification dudit peptide analogue.

**6.** Un analogue de la bradykinine ou son sel selon l'une quelconque des revendications 1 à 4, pour une utilisation en thérapie.

**7.** Un analogue de la bradykinine ou son sel selon l'une quelconque des revendications 1 à 4, pour une utilisation destinée' à soulager les états caractérisés par une dilatation ou une perméabilité vasculaire indésirables ou pour le traitement de la douleur ou de l'arthrite.

**8.** Un analogue de la bradykinine ou son sel selon l'une quelconque des revendications 1 à 4, pour une utilisation dans le traitement de l'hypertension, de l'insuffisance circulatoire ou de tout autre état dans lequel une augmentation du débit sanguin est nécessaire.

**9.** Utilisation d'un analogue de la bradykinine ou de son sel selon l'une quelconque des revendications 1 à 4, pour la fabrication d'un médicament destiné à être utilisé pour soulager dans des états caractérisés par une dilatation ou une perméabilité vasculaire indésirables, ou pour le traitement de la douleur ou de l'arthrite.

**10.** Utilisation d'un analogue de la bradykinine ou de son sel selon l'une quelconque des revendications 1 à 4, pour la fabrication d'un médicament destiné à être utilisé dans le traitement de l'hypertension, de l'insuffisance circulatoire ou de tout état dans lequel une augmentation du débit sanguin est nécessaire.